# EUROPÄISCHE PATENTANMELDUNG

(11) **EP 2 237 572 A1**
(43) Veröffentlichungstag der Anmeldung: **06.10.2010**
(21) Anmeldenummer: 10153602.7
(22) Anmeldetag: 15.02.2010
(51) Int. Cl.: H04R 25/00

(54) **Verfahren zum lautheitsbasierten Einstellen der Verstärkung eines Hörgeräts und zugehöriges Hörgerät**

(30) Priorität: 02.04.2009 DE 102009016058; 25.06.2009 DE 102009030551
(71) Anmelder: Siemens Medical Instruments Pte. Ltd., Singapore 139959 (SG)
(72) Erfinder: Latzel, Matthias, 91330 Eggolsheim (DE)
(74) Vertreter: Maier, Daniel Oliver

(57) **Zusammenfassung**

Die Erfindung gibt ein Verfahren und ein zugehöriges Hörgerät zum lautheitsbasierten Einstellen der Verstärkung des Hörgeräts (1, LH, RH) durch Darbieten von Testsignalen (TS) eines vorgebbaren Pegels und einer vorgebbaren Frequenz an. Vor und zwischen den Testsignalen (TS) werden Blindsignale (BS) dargeboten, wobei die Blindsignale (BS) zum Einstellen der Verstärkung des Hörgeräts (1, LH, RH) bei dem vorgegebenen Pegel und der vorgegebenen Frequenz nicht berücksichtigt werden. Ein Verfahren zum binauralen lautheitsbasierten Einstellen der Verstärkung eines linken und rechten Hörgeräts (1, LH, RH) wird ebenfalls angegeben. Vorteilhaft daran ist, dass es einem Hörgeräteträger leichter fällt, die dargebotenen Testsignale in seinem individuellen Lautheitswertesystem einzuordnen und entsprechend zu beurteilen.

## Beschreibung

Die Erfindung betrifft in den Patentansprüchen 1 und 8 angegebene Verfahren zur lautheitsbasierten Einstellung der Verstärkung eines Hörgeräts durch Darbieten von Testsignalen und in den Patentansprüchen 10 und 12 angegebene Hörgeräte.

Die zum Ausgleich eines Hörverlustes optimale Verstärkung eines Hörgeräts wird üblicherweise durch Lautheitsskalierungen oder hörschwellenbasierte präskriptive Anpassformeln bestimmt. Die Anpassung von Hörgeräten mittels Lautheitsskalierungen ist ein aufwändiger Vorgang und führt nicht immer zu optimalen Einstellungen. Daher gibt es Verfahren, bei denen ein Träger des Hörgeräts in die Anpassung interaktiv mit einbezogen wird. Dadurch wird die Anpasszeit verringert und vereinfacht sowie die Einstellung in strukturierter Weise optimiert.

Die Durchführung derartiger Verfahren erfolgt entweder bei einem Hörgeräteakustiker in einer simulierten akustischen Umgebung oder aber in der alltäglichen Umgebung des Hörgeräteträgers, wobei das Hörgerät angepasst wird und danach im Alltag erprobt werden kann.

Eine bekanntes adaptives Verfahren zur Anpassung eines Hörgeräts ist das "ScalAdapt-Verfahren", das bereits in den 90er Jahren des vorigen Jahrhunderts in Kiessling J. et al., "Adaptive Fitting of hearing instruments by category loudnes scaling (ScalAdapt)", Scand Audiol 1996; 25; 153-60 beschrieben wurde. Während in der beschriebenen Version die Präsentation der Einstellsignale im Freifeld erfolgte, wurden in einer kommerziellen Version die Testsignale direkt im Hörgerät erzeugt und einem Hörgeräteträger präsentiert. Das Verfahren konnte sich jedoch am Markt nicht durchsetzen, da das Verfahren nicht immer zu einer optimalen Einstellung der Hörgeräte führte.

Es ist Aufgabe der Erfindung ein verbessertes Verfahren und ein verbessertes Hörgerät zur Einstellung der Verstärkung des Hörgeräts in Abhängigkeit des Hörvermögens eines Hörgeräteträgers anzugeben.

Gemäß der Erfindung wird die gestellte Aufgabe mit den Verfahren der unabhängigen Patentansprüche 1 und 8 sowie den Hörgeräten der unabhängigen Patentanspruchs 10 und 12 gelöst.

Die grundsätzliche Idee der Erfindung besteht darin, bei einer lautheitsbasierten Anpassung der Verstärkung von Hörgeräten zusätzlich zu Testsignalen Blindsignale ("Dummy-Präsentationen") darzubieten, die für die Anpassung der Verstärkung nicht verwendet werden. Zusätzlich können die Frequenzen und Pegel der Blindsignale zufallsverteilt sein. Eine Lautheitsbeurteilung der Blindsignale kann für nachfolgende Anpassungen und Anpassschritte verwendet werden. Diese werden daher zwischengespeichert.

Die Erfindung beansprucht ein Verfahren zum lautheitsbasierten Einstellen der Verstärkung eines Hörgeräts durch Darbieten von Testsignalen eines vorgebbaren Pegels und einer vorgebbaren Frequenz. Zwischen den Testsignalen werden Blindsignale dargeboten, wobei die Blindsignale zum Einstellen der Verstärkung des Hörgeräts bei dem vorgegebenen Pegel und der vorgegebenen Frequenz nicht berücksichtigt werden. Die Blindsignale sind sogenannte "Dummy-Präsentationen". Vorteilhaft daran ist, dass es einem Hörgeräteträger leichter fällt, die dargebotenen Testsignale in seinem individuellen Lautheitswertesystem einzuordnen und entsprechend zu beurteilen.

In einer Weiterbildung können die Blindsignale unterschiedliche Pegel aufweisen. Der Ankerpunkt wird dadurch sicherer.

In einer weiteren Ausführungsform können die Blindsignale breitbandig sein.

Vorzugsweise können die Blindsignale schmalbandig sein und unterschiedliche Frequenzen haben.

Des Weiteren können die Pegel und/oder Frequenzen der Blindsignale zufallsabhängig eingestellt werden. Dies bietet den Vorteil, dass der absolute Anker bei der Anpassung nicht verloren geht und damit die Lautheitsbeurteilungen des Hörgeräteträgers genau bleiben. Dadurch wird eine Anpassung des Hörgeräts in eine falsche Richtung vermieden.

In einer Weiterbildung des Verfahrens kann eine Lautheitsbeurteilung der Blindsignale für die Einstellung des Hörgeräts bei weiteren vorgebbaren Pegeln und vorgebbaren Frequenzen in späteren Anpassschritten bzw. Anpassungen verwendet werden. Vorteilhaft daran ist, dass die Anpassung der Verstärkung für weitere Pegel und/oder Frequenzen beschleunigt wird.

In einer weiteren Ausführungsform können die Blind- und Testsignale im Hörgerät erzeugt werden. Vorteilhaft daran ist eine einfache Einstellung der Verstärkung von Hörgeräten.

Die Erfindung gibt auch ein Verfahren zum lautheitsbasierten, binauralen Einstellen der Verstärkung eines rechten und eines linken Hörgeräts an. Dabei wird zuerst die Verstärkung des rechten oder linken Hörgeräts nach dem erfindungsgemäßen, monauralen Verfahren eingestellt. Anschließend wird die Verstärkung des anderen Hörgeräts durch Darbieten von Testsignalen eingestellt. Vorteilhaft daran ist, dass die Verstärkung beider Hörgeräte optimal aufeinander abgestimmt wird.

In einer weiteren Ausführungsform kann das Einstellen der Verstärkung des anderen Hörgeräts beendet werden, wenn die verstärkten Testsignale des linken und des rechten Hörgeräts von einem Hörgerätenutzer als gleich laut wahrgenommen werden.

Die Erfindung gibt auch ein Hörgerät an, das die Blind- und Testsignale zur lautheitsbasierten Einstellung der Verstärkung eines Hörgeräts nach dem erfindungsgemäßen Verfahren erzeugt und entsprechend verstärkt abgibt.

In einer bevorzugten Ausführungsform kann das Hörgerät einem Rauschgenerator zur Erzeugung der Blind- und Testsignale umfassen.

Die Erfindung umfasst auch ein linkes und ein rechtes Hörgerät, welche die Blind- und Testsignale zur lautheitsbasierten Einstellung der Verstärkung des linken und rechten Hörgeräts nach dem erfindungsgemäßen Verfahren erzeugen und verstärkt abgeben.

In einer Weiterbildung können das linke und das rechte Hörgerät mit einem Rauschgenerator zur Erzeugung der Blind- und Testsignale ausgestattet sein.

Weitere Besonderheiten und Vorteile der Erfindung werden aus den nachfolgenden Erläuterungen mehrerer Ausführungsbeispiele anhand von schematischen Zeichnungen ersichtlich.

Es zeigen:
- Figur 1:: ein Ablaufdiagramm einer monauralen, schmalbandigen Anpassung eines Hörgeräts,
- Figur 2:: ein Ablaufdiagramm einer binauralen, schmalbandigen Anpassung eines Hörgeräts ,
- Figur 3:: ein Ablaufdiagramm einer monauralen, breitbandigen Anpassung eines Hörgeräts,
- Figur 4:: ein Ablaufdiagramm einer binauralen, breitbandigen Anpassung eines Hörgeräts und
- Figur 5:: eine Anordnung mit einem Hinter-dem-Ohr Hörgerät.

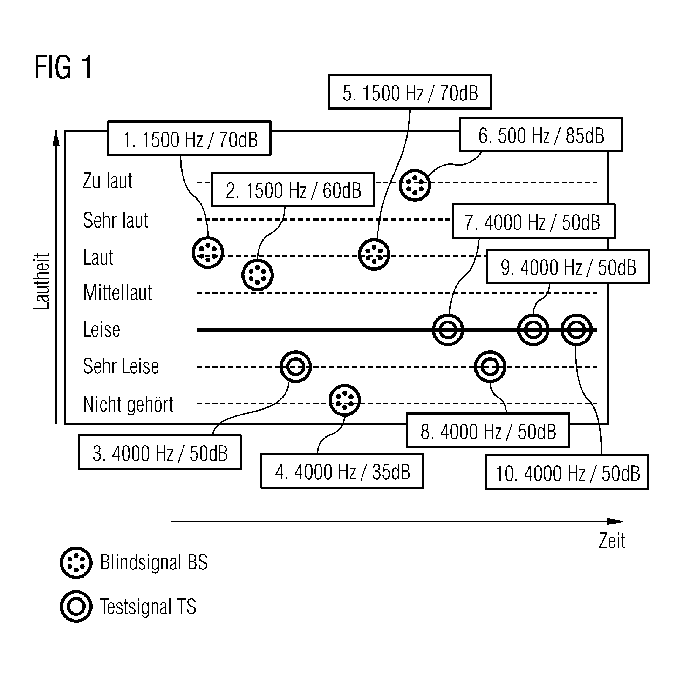

Figur 1 zeigt ein Ablaufdiagramm einer monauralen schmalbandigen Anpassung der Verstärkung eines Hörgeräts. Dazu wird beispielsweise im Hörgerät mittels eines Rauschgenerators ein Schmalbandrauschen entsprechend vorgebbarer Frequenzen und Pegel erzeugt. Die Frequenzen und Pegel können entsprechend nationaler oder internationaler Normen zur lautheitsbasierten Einstellung von Hörgeräten gewählt werden. Aus dem Schmalbandrauschen werden sowohl Blindsignale BS als auch Testsignale TS gebildet. Diese werden entsprechend der eingestellten Verstärkung des Hörgeräts verstärkt und einem Träger des Hörgeräts für eine Lautheitsbeurteilung dargeboten. Alternativ können die Blind- und Testsignale BS, TS auch von einer externen Quelle im Freifeld abgegeben werden.

Erfindungsgemäß werden nicht nur Testsignale TS, die für eine Anpassung der Verstärkung Verwendung finden, dargeboten, sondern zusätzlich auch Blindsignale BS, um dem Hörgeräteträger einen absoluten Ankerpunkt für eine Lautheitsorientierung zu geben. Die Pegel der sogenannten "Dummy-Darbietungen" liegen bevorzugt am unteren oder oberen Ende des Dynamikbereichs und werden zufällig gewählt. Dadurch fällt es dem Hörgeräteträger leichter, die dargebotenen Testsignale TS in seinem individuellen Lautheitswertesystem einzuordnen und entsprechend einer Skalierung von "nicht gehört" bis "zu laut" zu bewerten. Die Lautheitseinschätzungen der Blindsignale BS werden allerdings verworfen und gehen nicht in die Einstellung der Verstärkung ein.

Die Frequenzen der Blindsignale BS werden erfindungsgemäß zufällig gewählt, damit der absolute Anker während der Anpassung des Hörgeräts für den Hörgeräteträger nicht verloren geht.

Figur 1 zeigt beispielhaft die monaurale Anpassung eines Hörgeräts für ein schmalbandiges Testsignal TS von 4000 Hz und einem "leisen" Pegel von 50 dB. Die x-Richtung gibt die Zeit und die y-Richtung die Lautheitsbeurteilung durch den Hörgeräteträger an. Mit 1. bis 10. sind Anpassschritte bezeichnet.

Im 1. Schritt wird ein lautes Blindsignal BS mit 500 Hz Frequenz und 70 dB Schaldruckpegel als Anker dargeboten. Die Beurteilung "laut" geht nicht in die Einstellung der Verstärkung ein. Im 2. Schritt wird wiederum ein Blindsignal BS dargeboten, abweichend vom 1. Schritt mit einem Pegel von 60 dB und einer Frequenz von 1500 Hz. Der 3. Schritt präsentiert das erste Testsignal TS mit 4000 Hz Frequenz und 50 dB Pegel. Der Hörgeräteträger beurteilt das Testsignal TS des 3. Schritts als "sehr leise", so dass die Verstärkung des Hörgeräts erhöht wird. Im 4. Schritt erfolgt die Darbietung eines weiteren Blindsignals BS mit 4000 Hz und einem Pegel von 35 dB. Das Blindsignal des 4. Schritts wird vom Hörgeräteträger nicht gehört. Im folgenden 5. Schritt wird ein Blindsignal BS bei 1500 Hz und einem Pegel von 70 dB dargeboten. Der Hörgeräteträger stuft die Lautheit als "laut" ein. Im 6. Schritt wird ein weiteres Blindsignal BS dargeboten. Es hat einen Pegel von 85 dB und eine Frequenz von 500 Hz und wird vom Hörgeräteträger als "zu laut" eingestuft. In den folgenden vier Schritten wird jeweils das leise Testsignal TS mit der Frequenz 4000 Hz und dem Pegel 50 dB präsentiert. Im 7. Schritt wird der Testpegel TS als leise beurteilt, die Verstärkung wird daher konstant gehalten. Bei nochmaliger Präsentation im 8. Schritt wird das Testsignal TS als "sehr leise" eingestuft, so dass die Verstärkung des Hörgeräts erhöht wird. Im 9.und 10. Schritt wird das Testsignal TS mit "leise" beurteilt, so dass der Anpassvorgang beendet wird. Die Zielkategorie "leise" wurde zweimal hintereinander "getroffen". Die jeweils notwendige Änderung der Verstärkung wird aus dem Abstand der Beurteilung des Hörgerätenutzers zur Zielkategorie errechnet.

Nach dem erfolgreichen Einstellen der Verstärkung für die Lautheitskategorie "leise" wird die Verstärkung des Hörgeräts für eine andere Lautheitskategorie und eine andere Frequenz angepasst. Das Verfahren wird solange wiederholt, bis die Verstärkungen für alle vorgegebenen Lautheitskategorien und Frequenzen eingestellt sind.

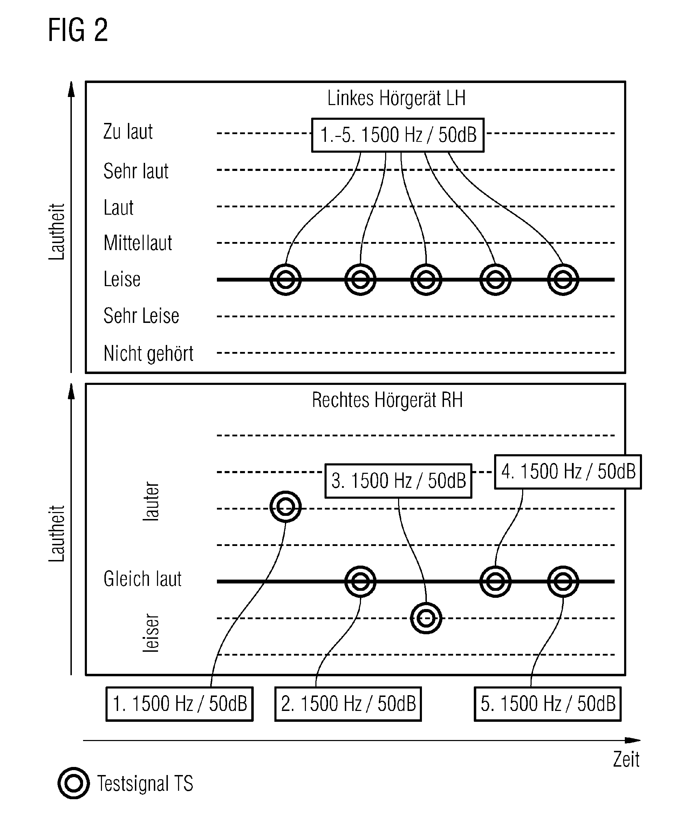

In Figur 2 ist beispielhaft die binaurale Anpassung eines rechten und eines linken Hörgeräts RH, LH für ein schmalbandiges Testsignal TS von 1500 Hz und einem "leisen" Pegel von 50 dB dargestellt. Die x-Achse und die y-Achse des Diagramms geben wiederum die Zeit bzw. die Lautheit an. Zuerst wird wie bei Figur 1 beschrieben das linke Hörgerät HG angepasst. Anschließend erfolgt eine gleichzeitige Präsentation von Testsignalen TS mit 1500 Hz und 50 dB durch das linke und rechte Hörgerät LH, RH. Der Hörgerätnutzer beurteilt, ob die Lautheit des rechten Hörgeräts RH "lauter" oder "leiser" als die Lautheit des linken Hörgeräts LH ist, oder ob beide Hörgeräte LH, RH "gleich laut" empfunden werden.

Die x-Richtung in Figur 2 gibt die Zeit und die y-Richtung die Lautheitsbeurteilung durch den Hörgeräteträger an Mit 1. bis 5. sind Einstellschritte bezeichnet. Im 1. Schritt wird die Lautheit des rechten Hörgeräts HG als "lauter" beurteilt, so dass die Verstärkung des rechten Hörgeräts HG verringert wird. Im 2. Schritt wird die Lautheit als "gleich laut" beurteilt, die Verstärkung wird konstant gehalten. Im folgenden 3. Schritt wird die Lautheit als "leiser" bewertet. Die Verstärkung wird erhöht. Im 4. und 5. Schritt wird die Lautheit als "gleich laut" beurteilt, so dass die Verstärkung konstant gehalten wird. Da beide Hörgeräte LH, RH zweimal hintereinander als "gleich laut" beurteilt werden, ist der Einstellvorgang erfolgreich beendet.

Nach dem erfolgreichen binauralen Anpassen der Verstärkung für die Lautheitskategorie "leise" und der Frequenz 1500 Hz wird die Verstärkung des rechten Hörgeräts für eine andere Lautheitskategorie und eine andere Frequenz angepasst. Das Verfahren wird solange wiederholt, bis die Verstärkungen des rechten Hörgeräts RH für alle vorgegebenen Lautheitskategorien und Frequenzen angepasst sind. Voraussetzung dafür ist, dass das linke Hörgerät LH für alle Frequenzen und Lautheiten eingestellt wurde.

Das Verfahren zur lautheitsbasierten binauralen Anpassung der Verstärkung kann selbstverständlich auch mit einer monauralen Anpassung des rechten Hörgeräts RH beginnen und anschließend wird das linke Hörgerät LH binaural eingestellt.

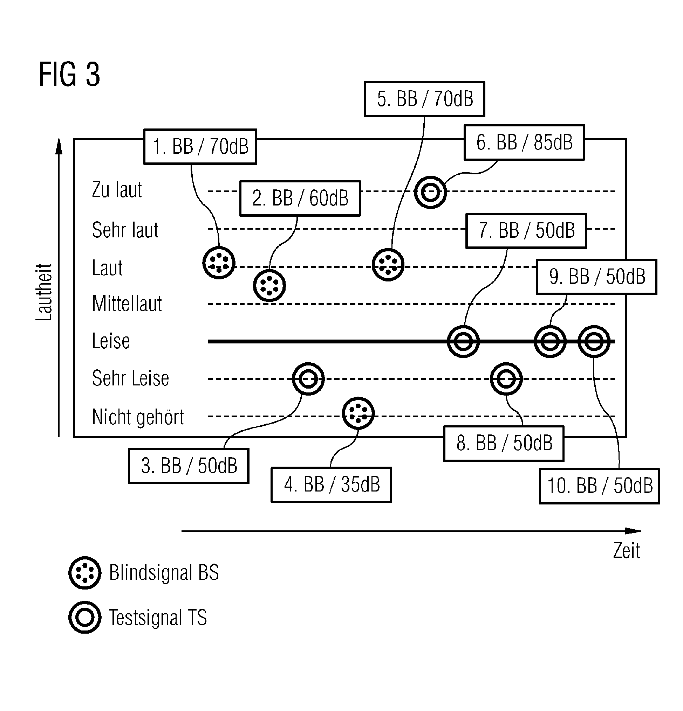

Figur 3 zeigt ein Ablaufdiagramm einer monauralen breitbandigen Anpassung der Verstärkung eines Hörgeräts. Dazu wird beispielsweise im Hörgerät selbst mittels eines Rauschgenerators ein Breitbandrauschen entsprechend vorgebbarer Pegel erzeugt. Die Pegel können gemäß nationaler oder internationaler Normen zur lautheitsbasierten Einstellung von Hörgeräten gewählt werden. Aus dem Breitbandrauschen werden sowohl Blindsignale BS als auch Testsignale TS gebildet. Diese werden entsprechend der eingestellten Verstärkung des Hörgeräts verstärkt und dem Träger des Hörgeräts für eine Lautheitsbeurteilung dargeboten. Alternativ können die Blind- und Testsignale BS, TS auch von einer externen Quelle abgegeben werden.

Erfindungsgemäß werden nicht nur Testsignale TS, die für eine Anpassung der Verstärkung Verwendung finden, dargeboten, sondern zusätzlich auch Blindsignale BS, um dem Hörgeräteträger einen absoluten Ankerpunkt für eine entsprechende Lautheitsorientierung zu geben. Die Pegel der sogenannten "Dummy-Darbietungen" liegen bevorzugt am unteren oder oberen Ende des Dynamikbereichs und werden zufällig gewählt. Dadurch fällt es dem Hörgeräteträger leichter, die dargebotenen Testsignale TS in seinem individuellen Lautheitswertesystem einzuordnen und gemäß einer Skalierung von "nicht gehört" bis "zu laut" zu bewerten. Die Lautheitseinschätzungen der Blindsignale BS werden allerdings verworfen und gehen nicht in die Einstellung der Verstärkung ein.

Figur 3 zeigt beispielhaft die monaurale Anpassung eines Hörgeräts für ein breitbandiges Testsignal TS mit einem "leisen" Pegel von 50 dB. Die x-Richtung gibt die Zeit und die y-Richtung die Lautheitsbeurteilung durch den Hörgeräteträger an. Mit 1. bis 10. sind Einstellschritte bezeichnet. Im 1. Schritt wird ein Blindsignal BS mit 70 dB Schalldruckpegels als Anker dargeboten. Die Beurteilung "laut" geht nicht in die Anpassung der Verstärkung ein. Im 2. Schritt wird wiederum ein Blindsignal BS dargeboten, abweichend vom 1. Schritt mit einem Pegel von 60 dB. Der 3. Schritt präsentiert das erste Testsignal TS mit 50 dB Pegel. Der Hörgeräteträger beurteilt das Testsignal TS des 3. Schritts als "sehr leise", so dass die Verstärkung des Hörgeräts erhöht wird. Im 4. Schritt erfolgt die Darbietung eines weiteren Blindsignals BS mit einem Pegel von 35 dB. Das Blindsignal des 4. Schritts wird vom Hörgeräteträger nicht gehört. Im folgenden 5. Schritt wird ein Blindsignal BS mit einem Pegel von 70 dB dargeboten. Der Hörgeräteträger stuft die Lautheit als "laut" ein. Im 6. Schritt wird ein weiteres Blindsignal BS dargeboten. Es hat einen Pegel von 85 dB und wird vom Hörgeräteträger als "zu laut" eingestuft. In den folgenden vier Schritten wird jeweils das Testsignal TS mit dem Pegel 50 dB präsentiert. Im 7. Schritt wird der Testpegel TS als "leise" beurteilt, die Verstärkung wird daher konstant gehalten. Bei nochmaliger Präsentation im 8. Schritt wird das Testsignal TS als "sehr leise" eingestuft, so dass die Verstärkung des Hörgeräts erhöht wird. Im 9.und 10. Schritt wird das Testsignal TS mit "leise" beurteilt, so dass der Einstellvorgang beendet wird. Die Zielkategorie "leise" wurde zweimal hintereinander "getroffen". Die jeweils notwendige Änderung der Verstärkung wird aus dem Abstand der Beurteilung des Hörgerätenutzers zur Zielkategorie errechnet.

Nach dem erfolgreichen Einstellen der Verstärkung für die Lautheitskategorie "leise" wird die Verstärkung des Hörgeräts für eine andere Lautheitskategorie angepasst. Das Verfahren wird solange wiederholt, bis die Verstärkungen für alle vorgegebenen Lautheitskategorien eingestellt sind.

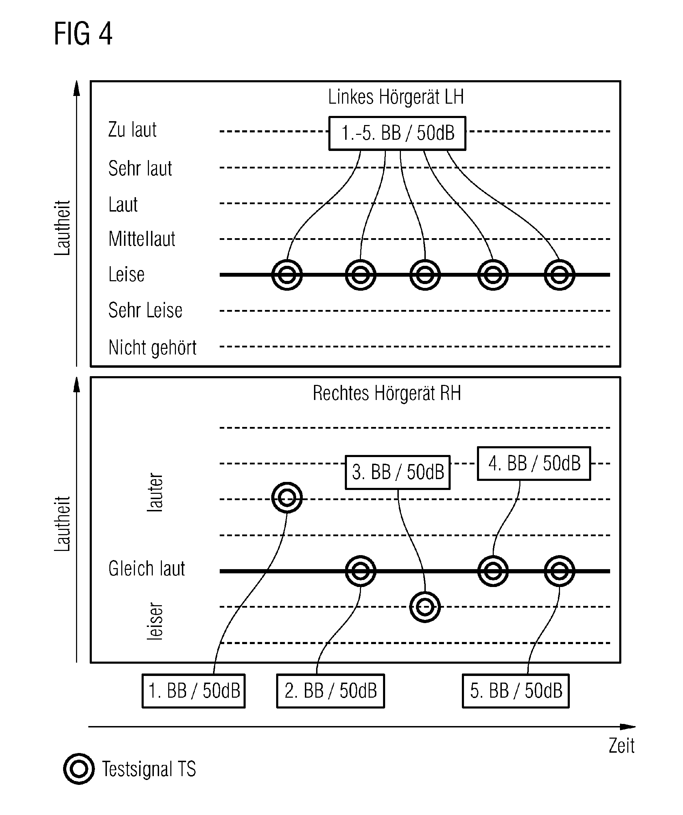

In Figur 4 ist beispielhaft die binaurale Anpassung eines rechten und eines linken Hörgeräts RH, LH für ein breitbandiges Testsignal TS mit einem "leisen" Pegel von 50 dB dargestellt. Die x-Achse und die y-Achse des Diagramms geben wiederum die Zeit bzw. die Lautheit an. Zuerst wird wie bei Figur 3 beschrieben das linke Hörgerät HG angepasst. Anschließend erfolgt eine gleichzeitige Präsentation von Testsignalen TS mit 50 dB durch das linke und rechte Hörgerät LH, RH. Der Hörgerätnutzer beurteilt, ob die Lautheit des rechten Hörgeräts RH "lauter" oder "leiser" als die Lautheit des linken Hörgeräts LH ist, oder ob beide Hörgeräte LH, RH "gleich laut" klingen.

Die x-Richtung in Figur 4 gibt die Zeit und die y-Richtung die Lautheitsbeurteilung durch den Hörgeräteträger an. Mit 1. bis 5. sind Anpassschritte bezeichnet. Im 1. Schritt wird die Lautheit des rechten Hörgeräts RH als "lauter" beurteilt, so dass die Verstärkung des rechten Hörgeräts RH verringert wird. Im 2. Schritt wird die Lautheit als "gleich laut" beurteilt, die Verstärkung wird konstant gehalten. Im folgenden 3. Schritt wird die Lautheit als "leiser" bewertet. Die Verstärkung wird erhöht. Im 4. und 5. Schritt wird die Lautheit als "gleich laut" beurteilt, so dass die Verstärkung konstant gehalten wird. Da beide Hörgeräte LH, RH zweimal hintereinander als "gleich laut" beurteilt werden, ist der Einstellvorgang erfolgreich beendet.

Nach dem erfolgreichen binauralen Anpassen der Verstärkung für die Lautheitskategorie "leise" wird die Verstärkung des rechten Hörgeräts für eine andere Lautheitskategorie eingestellt. Das Verfahren wird solange wiederholt, bis die Verstärkungen des rechten Hörgeräts RH für alle vorgegebenen Lautheitskategorien eingestellt sind.

Das Verfahren zur lautheitsbasierten binauralen Anpassung der Verstärkung kann selbstverständlich auch mit einer monauralen Anpassung des rechten Hörgeräts RH beginnen und anschließend das linke Hörgerät LH binaural angepasst werden.

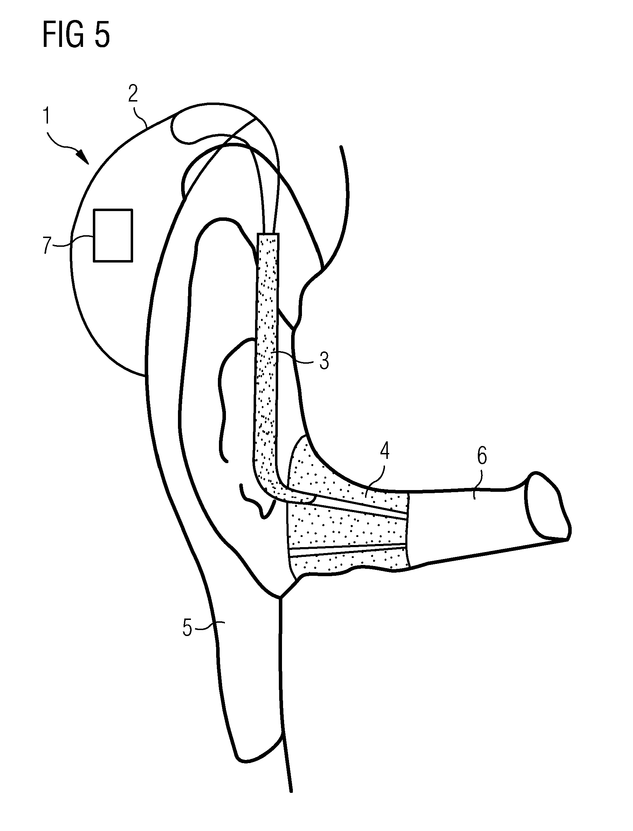

In Figur 5 ist ein erfindungsgemäßes Hinter-dem-Ohr Hörgerät 1 mit einem Hörgerätegehäuse 2, einem Schallschlauch 3 und einer Otoplastik 4 schematisch dargestellt. Das Hörgerätegehäuse 2 sitzt hinter dem Ohr 5 eines Hörgeräteträgers. Das Hörgerätegehäuse 2 ist mit dem Schallschlauch 3 verbunden. Der Schallschlauch 3 schließt mit der Otoplastik 4 ab, die den Schallschlauch 3 im Gehörgang 6 des Hörgerätenutzers fixiert. Im Hörgerätegehäuse 2 ist ein Rauschgenerator 7 angeordnet, der schmalbandige und breitbandige Rauschsignale erzeugt, die für die Darbietung von Blind- und Testsignalen nach den Verfahren der Figuren 1 bis 4 verwendet werden.

Für eine binaurale Kopplung des linken und des rechten Hörgeräts LH, RH können die beiden Hörgeräte LH, RH Daten per Funkkommunikation austauschen.

Lautheitsbeurteilungen von Blindsignalen bei einer bestimmten Frequenz und/oder einem bestimmten Pegel können für eine vereinfachte Anpassung der Verstärkung bei einem anderen Pegel und/oder einer anderen Frequenz verwendet werden.

## Patentansprüche

1. Verfahren zum lautheitsbasierten Einstellen der Verstärkung eines Hörgeräts (1, LH, RH) durch Darbieten von Testsignalen (TS) eines vorgebbaren Pegels und einer vorgebbaren Frequenz,
**gekennzeichnet durch**:
- Darbieten von Blindsignalen (BS) vor und/oder zwischen den Testsignalen (TS), wobei die Blindsignale (BS) zum Einstellen der Verstärkung des Hörgeräts (1, LH, RH) bei dem vorgegebenen Pegel und der vorgegebenen Frequenz nicht berücksichtigt werden.

2. Verfahren nach Anspruch 1,
**dadurch gekennzeichnet,**
**dass** die Blindsignale (BS) unterschiedliche Pegel aufweisen.

3. Verfahren nach Anspruch 1 oder 2,
**dadurch gekennzeichnet,**
**dass** die Blindsignale (BS) breitbandig sind.

4. Verfahren nach Anspruch 1 oder 2,
**dadurch gekennzeichnet,**
**dass** die Blindsignale (BS) schmalbandig sind und unterschiedliche Frequenzen aufweisen.

5. Verfahren nach einem der vorhergehenden Ansprüche,
**dadurch gekennzeichnet,**
**dass** die Pegel und/oder Frequenzen der Blindsignale (BS) zufallsabhängig gewählt werden.

6. Verfahren nach einem der vorhergehenden Ansprüche,
**dadurch gekennzeichnet,**
**dass** eine Lautheitsbeurteilung der Blindsignale (BS) für die Einstellung des Hörgeräts (1, LH, RH) bei weiteren vorgebbaren Pegeln und vorgebbaren Frequenzen verwendet wird.

7. Verfahren nach einem der vorhergehenden Ansprüche,
**dadurch gekennzeichnet,**
**dass** die Blind- und Testsignale (B, TS) im Hörgerät (1, LH, RH) erzeugt werden.

8. Verfahren zum lautheitsbasierten, binauralen Einstellen der Verstärkung eines rechten und eines linken Hörgeräts (RH, LH),
**gekennzeichnet durch**:
- Einstellen der Verstärkung des rechten oder linken Hörgeräts (RH oder LH) nach dem Verfahren nach einem der vorhergehenden Ansprüche und
- Einstellen der Verstärkung des anderen Hörgeräts (LH oder RH) **durch** Darbieten von Testsignalen (TS).

9. Verfahren nach Anspruch 8,
**dadurch gekennzeichnet,**
**dass** das Einstellen der Verstärkung des anderen Hörgeräts (LH oder RH) beendet wird, wenn die verstärkten Testsignale (TS) des linken und des rechten Hörgeräts (LH, RH) von einem Hörgerätenutzer als gleich laut wahrgenommen werden.

10. Hörgerät (1, LH, RH), das die Blind- und Testsignale (BS; TS) zum lautheitsbasierten Einstellen der Verstärkung eines Hörgeräts (1, LH, RH) nach einem der Ansprüche 1 bis 7 erzeugt und verstärkt abgibt.

11. Hörgerät (1, LH, RH) nach Anspruch 10 mit einem Rauschgenerator (7) zur Erzeugung der Blind- und Testsignale (BS, TS).

12. Linkes und rechtes Hörgerät (LH, RH), die die Blind- und Testsignale (BS, TS) zum lautheitsbasierten Einstellen der Verstärkung des linken und rechten Hörgeräts (LH, RH) nach Anspruch 8 oder 9 erzeugen und verstärkt abgeben.

13. Linkes und rechtes Hörgerät (LH, RH) nach Anspruch 12 mit einem Rauschgenerator (7) zur Erzeugung der Blind- und Testsignale (BS, TS).

## Geänderte Patentansprüche

### Geänderte Patentansprüche gemäss Regel 137(2) EPÜ.

**1.** Verfahren zum lautheitsbasierten Einstellen der Verstärkung eines Hörgeräts (1, LH, RH) durch Darbieten von Testsignalen (TS) eines vorgebbaren Pegels und einer vorgebbaren Frequenz,
**gekennzeichnet durch**:
- Darbieten von hörbaren und unhörbaren Blindsignalen (BS) vor und/oder zwischen den Testsignalen (TS), wobei die Blindsignale (BS) unterschiedliche Pegel aufweisen und die Blindsignale (BS) zum Einstellen der Verstärkung des Hörgeräts (1, LH, RH) bei dem vorgegebenen Pegel und der vorgegebenen Frequenz nicht berücksichtigt werden.

**2.** Verfahren nach Anspruch 1,
**dadurch gekennzeichnet,**
**dass** die Blindsignale (BS) breitbandig sind.

**3.** Verfahren nach Anspruch 1,
**dadurch gekennzeichnet,**
**dass** die Blindsignale (BS) schmalbandig sind und unterschiedliche Frequenzen aufweisen.

**4.** Verfahren nach einem der vorhergehenden Ansprüche,
**dadurch gekennzeichnet,**
**dass** die Pegel und/oder Frequenzen der Blindsignale (BS) zufallsabhängig gewählt werden.

**5.** Verfahren nach einem der vorhergehenden Ansprüche,
**dadurch gekennzeichnet,**
**dass** eine Lautheitsbeurteilung der Blindsignale (BS) zum lautheitsbasierten Einstellen der Verstärkung des Hörgeräts (1, LH, RH) bei weiteren vorgebbaren Pegeln und vorgebbaren Frequenzen ausgeführt wird.

**6.** Verfahren nach einem der vorhergehenden Ansprüche,
**dadurch gekennzeichnet,**
**dass** die Blind- und Testsignale (B, TS) im Hörgerät (1, LH, RH) erzeugt werden.

**7.** Verfahren zum lautheitsbasierten, binauralen Einstellen der Verstärkung eines rechten und eines linken Hörgeräts (RH, LH),
**gekennzeichnet durch**:
- Einstellen der Verstärkung des rechten oder linken Hörgeräts (RH oder LH) nach dem Verfahren nach einem der vorhergehenden Ansprüche und
- Einstellen der Verstärkung des anderen Hörgeräts (LH oder RH) **durch** Darbieten von Testsignalen (TS).

**8.** Verfahren nach Anspruch 7,
**dadurch gekennzeichnet,**
**dass** das Einstellen der Verstärkung des anderen Hörgeräts (LH oder RH) beendet wird, wenn die durch das linke und rechte Hörgerät (RH, LH) dargebotenen Testsignale (TS) von einem Hörgerätenutzer als gleich laut wahrgenommen werden.

**9.** Hörgerät (1, LH, RH),
**gekennzeichnet durch**:
- einen Rauschgenerator (7) zur Erzeugung von Blind- und Testsignalen (BS, TS), der die Blind- und Testsignale (BS; TS) zum lautheitsbasierten Einstellen der Verstärkung des Hörgeräts (1, LH, RH) nach einem der Ansprüche 1 bis 6 erzeugt und abgibt.

**10.** Linkes und rechtes Hörgerät (LH, RH), **gekennzeichnet durch**:
- jeweils einen Rauschgenerator (7) zur Erzeugung von Blind- und Testsignalen (BS, TS), der die Blind- und Testsignale (BS, TS) zum lautheitsbasierten Einstellen der Verstärkung des linken und rechten Hörgeräts (LH, RH) nach Anspruch 7 oder 8 erzeugt und abgibt.
